(19) Europäisches Patentamt
European Patent Office
Office européen des brevets

(11) Publication number: **0 102 163 B1**

(12) **EUROPEAN PATENT SPECIFICATION**

(45) Date of publication of patent specification: **01.04.87**

(21) Application number: **83304080.1**

(22) Date of filing: **13.07.83**

(51) Int. Cl.$^4$: **C 07 D 249/08,** C 07 D 233/60, A 01 N 43/64, A 01 N 43/50

# (54) Triazole derivatives and compositions containing the same.

(30) Priority: **14.07.82 US 398048**

(43) Date of publication of application: **07.03.84 Bulletin 84/10**

(45) Publication of the grant of the patent: **01.04.87 Bulletin 87/14**

(84) Designated Contracting States: **BE CH DE FR GB IT LI NL**

(58) References cited:
**EP-A-0 007 506**
**US-A-3 852 301**

(73) Proprietor: **CHEVRON RESEARCH COMPANY
525 Market Street
San Francisco California 94105 (US)**

(72) Inventor: **Cherpeck, Richard E.
40 Oxford Drive
San Rafael California 94903 (US)**

(74) Representative: **Kosmin, Gerald Emmanuel et al
HASELTINE, LAKE & CO. Hazlitt House
28 Southampton Buildings Chancery Lane
London, WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 102 163 B1

**Description**

This invention relates to novel triazole derivatives having utility as fungicides and plant growth regulators, and to fungicidal compositions containing them.

The compounds and compositions of the invention are especially effective against powdery mildew fungal infections. Moreover, at higher concentrations, the compounds and compositions exhibit advantageous plant growth regulating activity.

The compounds of the present invention can be represented by the general formula:

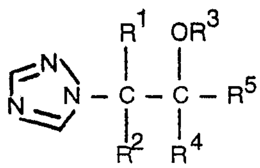

I

wherein

$R^1$ is isopropoxycarbonyl, t-butoxycarbonyl, or neopentoxycarbonyl;

$R^2$ is hydrogen, methyl or ethyl;

$R^3$ and $R^4$ are hydrogen; and

$R^5$ is 2,4-dihalophenyl,

with the proviso that when $R^1$ is neopentoxycarbonyl, $R^2$ is hydrogen.

Since the compounds of formula I have an asymmetric carbon atom, the compounds may exist as optical isomers. The above formula I is intended to represent both the respective optical isomers and mixtures thereof as well as geometric isomers, and such isomers and isomer mixtures are encompassed within the invention.

The invention also provides processes and intermediates for preparing the above compounds.

In a further aspect, the invention also provides fungicidal compositions comprising an inert compatible carrier and a fungicidally effective amount of a compound of formula I.

In another aspect, the invention also provides a method of controlling fungi causing fungal plant diseases which comprises applying an amount of a compound of formula I, effective to prevent or arrest the growth of fungi, to such fungi or to the potential growth medium of such fungi (e.g. vegetation).

In another aspect, the invention also provides a plant growth regulating composition comprising an inert compatible carrier and an amount of a compound of formula I effective to advantageously alter the growth pattern of plants.

In still another aspect, the invention also provides a method of regulating plant growth which comprises treating the growth medium and/or the foliage of plants with an amount of a compound of formula I effective to advantageously alter the growth pattern of such plants.

$R^5$ is preferably 2,4-dichlorophenyl.

The compounds of the present invention can be conveniently prepared according to the following overall reaction scheme:

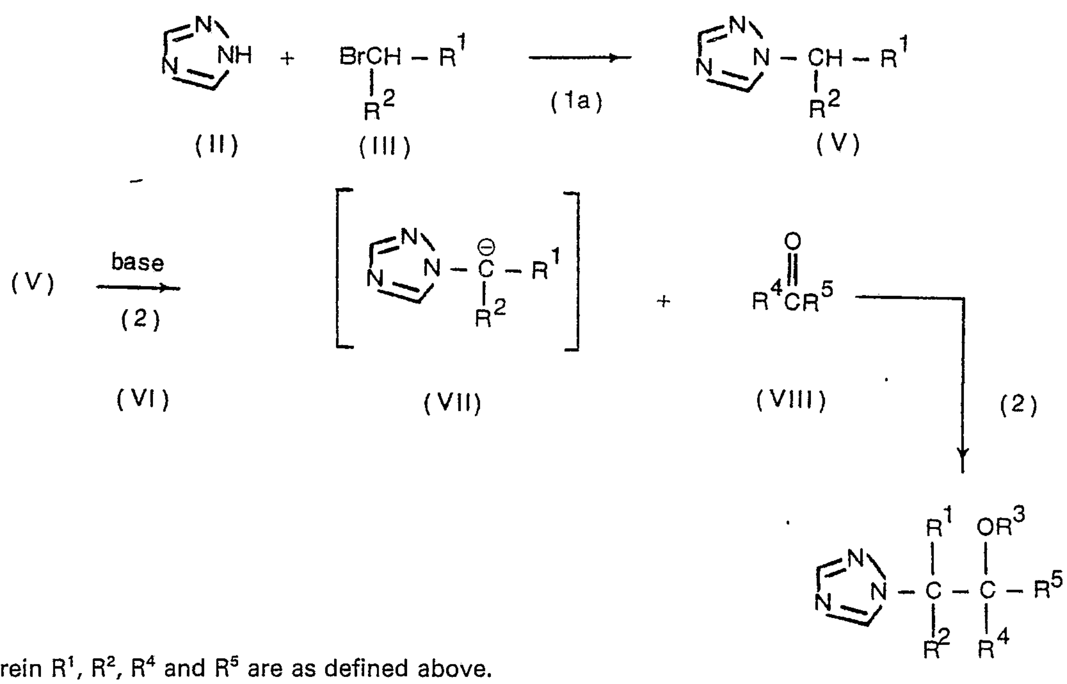


(II) (III) (V) (1)

(V) (VI) (VII) (VIII) (2)

I

wherein $R^1$, $R^2$, $R^4$ and $R^5$ are as defined above.

Step (1) can be conducted by contacting the appropriate triazole II with the appropriate starting material III having the desired $R^1$ and $R^2$ substitution under substantially anhydrous conditions preferably in an inert organic solvent in the presence of a scavenger base. Typically, this process is conducted at temperatures in the range of from −20° to 80°C, preferably 0° to 30°C, for from 1 to 72 hours. Generally, from 0.5 to 5 moles, preferably from 1 to 1.2 moles, of compound III are used per mole of compound II. Preferably, a base is added to the system to scavenge the acid generated during the reaction. Preferably an amine type base should not be used to avoid conflicting reactions. Suitable bases which can be used include, for example, alkali metal alkoxides, for example, sodium methoxide.

Suitable inert organic solvents which can be used include, for example, lower alkanols (e.g. methanol, ethanol), tetrahydrofuran, dimethylformamide, and dimethyl sulfoxide, and compatible mixtures thereof. Preferably, triazole isomerization is minimized in Reaction (1) by using ethanol as the solvent, sodium ethoxide as the base, and the bromoalkyl reagent, III. The product, V, can be isolated by suitable procedures such as extraction, filtration, chromatography, or distillation or alternatively is used directly in Reaction (2) without purification and/or isolation. The starting materials II and III are generally known compounds and can be prepared by known procedures or by obvious modifications thereof (e.g. substitution of appropriate substituents or solvents.

Step (2) can be effected by contacting intermediate V with compound VIII having the appropriate $R^4$ and $R^5$ substituents in the presence of a base under anhydrous conditions, preferably in an inert organic solvent and under an inert atmosphere. This process is typically conducted at temperatures in the range of from −50° to 100°C, preferably from −50° to 0°C, for from ¼ to 5 hours, using from 0.5 to 5 moles, preferably 0.9 to 1.2 moles, of compound VIII per compound V. The purpose of the base is to generate the anion of compound V shown as VII in the reaction equation which in turn reacts with compound VIII. Thus, in practice it is preferred to contact compound V with the base prior to the addition of compound VIII. Generally, about from 1 to 3 moles of base are used per mole of compound V and preferably about stoichiometric equivalent amounts of the base (preferably, a strong base such as lithium diethylamide or lithium diisopropylamide). Other bases which can be used include, for example, alkali metal t-butoxides and magnesium amides.

Suitable inert organic solvents which can be used include, for example, dioxane, tetrahydrofuran, dimethoxyethane and alkyl ethers (e.g. diethyl ether) and compatible mixtures thereof. Preferably, this process is conducted under essentially anhydrous conditions and under an inert atmosphere, conveniently under nitrogen or argon. Also, it is preferred to add the reagents at the lower temperatures within the above temperature range. The reaction is generally complete from within 1 to 24 hours. The product, I, can then be isolated by suitable procedures such as, for example, extraction, filtration, chromatography, or distillation.

Generally, the reactions described above are conducted as liquid-phase reactions and hence pressure is generally not significant except as it affects temperature (boiling point) where reactions are conducted at reflux. Therefore, these reactions are generally conducted at pressures of from 300 to 3000 mm mercury and conveniently are conducted at about atmospheric or ambient pressure.

It should also be appreciated that where typical or preferred process conditions (e.g. reaction temperatures, times, mole ratios of reactants and solvents) have been given, that conditions above or below these ranges may also be used in some instances but generally with poorer results or economies. Optimum conditions may vary with the particular reagents or organic solvents used but can be determined by routine optimization procedures.

Where optical isomer mixtures are obtained, the respective optical isomers can be obtained by conventional resolution procedures, for example, by reacting the isomer mixture with an optically active acid which will yield a mixture of optical salts, of the desired compound, which can be resolved by conventional procedures (e.g. crystallization) into the respective plus and minus optical salts.

*Utility*

The compounds of the present invention are effective in controlling plant fungal infections. Additionally, the compounds of the invention are particularly effective against powdery mildew fungal infections caused by such organisms as *Erysiphe polygoni*. Certain of the compounds of this invention are also useful in controlling leaf blights caused by organisms such as *Phytophthora infestans, Septoria apii,* and *Alternaria solani conidia* and/or controlling fungal infections caused by *Plasmopara viticola, Uromyces phaseoli tipica,* and *Piricularia oryzae*. Table II lists a summary of activity against some particular fungi for several compounds of this invention.

When used as fungicides, the compounds of the invention are applied in fungicidally effective amounts to fungi and/or their habitats, such as vegetative hosts. The amount used will, of course, depend on several factors such as the host, the type of fungus and the particular compound of the invention. As with most pesticidal compounds, the fungicides of the invention are not usually applied full strength, but are generally incorporated with conventional, biologically inert extenders or carriers normally employed for facilitating dispersion of active fungicidal compounds, recognizing that the formulation and mode of application may affect the activity of the fungicide. Thus, the fungicides of the invention may be formulated and applied as granules, as powdery dusts, as wettable powders, as emulsifiable concentrates, as

solutions, or as any of several other known types of formulations, depending on the desired mode of application.

Wettable powders are in the form of finely divided particles which disperse readily in water or other dispersant. These compositions normally contain from about 5% to 80% fungicide, and the rest inert material, which includes dispersing agents, emulsifying agents and wetting agents. The powder may be applied to the soil as a dry dust, or preferably as a suspension in water. Typical carriers include fuller's earth, kaolin clays, silicas, and other highly absorbent, readily wettable, inorganic diluents. Typical wetting, dispersing or emulsifying agents include, for example: the aryl and alkylaryul sulfonates and their sodium salts; alkylamide sulfonates, including fatty methyl taurides; alkylaryl polyether alcohols, sulfated higher alcohols, and polyvinyl alcohols; polyethylene oxides, sulfonated animal and vegetable oils; sulfonated petroleum oils, fatty acid esters of polyhydric alcohols and the ethylene oxide addition products of such esters; and the addition products of long-chain mercaptans and ethylene oxide. Many other types of useful surface-active agents are available in commerce. The surface-active agent, when used, normally comprises from 1% to 15% by weight of the fungicidal composition.

Dusts are freely flowing admixtures of the active fungicide with finely divided solids such as talc, natural clays, kieselguhr, pyrophyllite, chalk, diatomaceous earths, calcium, phosphates, calcium and magnesium carbonates, sulfur, lime, flours, and other organic and inorganic solids which act as dispersants and carriers for the toxicant. These finely divided solids have an average particle size of less than about 50 microns. A typical dust formulation useful herein contains 75% silica and 25% of the toxicant.

Useful liquid concentrates include the emulsifiable concentrates, which are homogenous liquid or paste compositions which are readily dispersed in water or other dispersant, and may consist entirely of the fungicide with a liquid or solid emulsifying agent, or may also contain a liquid carrier such as xylene, heavy aromatic naphthas, isophorone, and other nonvolatile organic solvents. For application, these concentrates are dispersed in water or other liquid carrier, and are normally applied as a spray to the area to be treated.

Other useful formulations for fungicidal applications include simple solutions of the active fungicide in a dispersant in which it is completely soluble at the desired concentration, such as acetone, alkylated naphthalenes, xylene, or other organic solvents. Granular formulations, wherein the fungicide is carried on relatively coarse particles, are of particular utility for aerial distribution or for genetration of cover-crop canopy. Pressurized sprays, typically aerosols wherein the active ingredient is dispersed in finely divided form as a result of vaporization of a low-boiling dispersant solvent carrier, such as the Freons, may also be used. All of those techniques for formulating and applying fungicides are well known in the art.

The percentages by weight of the fungicide may vary according to the manner in which the composition is to be applied and the particular type of formulation, but in general comprise 0.05% to 95% of the toxicant by weight of the fungicidal composition, and depending on whether the composition is intended for direct application or dilution prior to application. The compounds are typically applied at rates in the range of about from 0.1 to 5 kg/hectare, preferably 0.2 to 3 kg/hectare, and typically are applied as foliage sprays.

The fungicidal compositions may be formulated and applied with other active ingredients, including other fungicides, insecticides, nematocides, bactericides, plant growth regulators, fertilizers, etc.

The compound of the invention also exhibit plant growth regulating activity, including general yield enhancement and also stunting activity against grass crops. This stunting activity is particularly desirable in the case of crops such as wheat, since it reduces matting caused by the tall stems intertwining, without adversely affecting the amount of grain yielded, and in some instances even improves the yield.

The present compounds of formula I can be applied in pure form, but more pragmatically are applied in combination with a carrier. Depending on the desired application, the plant growth regulating composition can also contain, or be applied in combination with other compatible ingredients such as desiccants, defoliants, surface-active agents, adjuvants, fungicides, insecticides and selective herbicides. Typically, the plant growth regulating composition will contain a total of about from 0.1 to 95 weight percent, of one or more compounds of formula I, depending on whether the composition is designed for direct use or dilution prior to application. Generally, for plant growth regulating activity, the compounds of formula I will be applied at a dosage of about from 1 to 10 kg/ha, depending on the mode of application, plant, and type of plant growth regulating effect desired. The compounds are preferably applied as a seed pretreatment or soil drench as the compounds are best absorbed through the roots of the plant. As a seed pretreatment for yield enhancement, the compounds are generally preferably applied at a dosage of about 2 to 16 gms per 100 kg of seeds. As a soil drench, the compounds are generally preferably applied at a rate of about from 1 to 2.5 kg/hectare. At these soil drench rates, the compounds are generally ineffective as foliage sprays.

A further understanding of the invention can be had from the following non-limiting Examples wherein, unless expressly stated to the contrary, all temperatures and temperature ranges refer to the centigrade system and the term "ambient" or "room temperature" refers to about 20° to 25°C. The term "percent" refers to weight percent and the term "mole" or "moles" refers to gram moles. The term "equivalent" refers to a reagent equal in moles, to the moles of the preceding or succeeding reactant recited in that example in terms of finite moles or finite weight or volume. Unless expressly stated to the contrary, E and Z isomers are generated where appropriate and are not separated. Also, unless expressly

stated to the contrary, geometric isomer and racemic mixtures are used as starting materials and corresponding isomer mixtures are obtained as products.

## EXAMPLES

### Example 1

t-butyl α-(1,2,4-triazol-1-yl)acetate

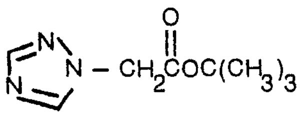

34.53 gms of 1,2,4-triazole were added to 250 mls of ethanol along with 34.0 gms of sodium ethoxide. The system was placed under an argon atmosphere and cooled to about 0°C. After cooling, 107.28 gms of t-butyl bromoacetate were dropwise added to the system. The system was then allowed to come to room temperature and stirred there for 60 hours. The reaction was stopped and the system filtered. The solvent was removed by stripping and the residue was dissolved in hot ether and then filtered. The ether was removed by stripping and the resulting solid triturated with hexane and dried to yield 80.81 gms of the title compound as a beige solid.

### Example 2

t-butyl α-(1,2,4-triazol-1-yl)-β-hydroxy-β-(2,4-dichlorophenyl)propionate

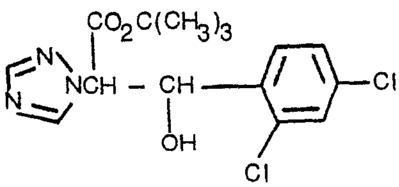

To a 3-neck, 250-ml flask equipped with an addition funnel, argon inlet and a septum were added to 60 mls of anhydrous tetrahydrofuran and 7.5 mls of diisopropylamine (passed through activity I neutral alumina) under an argon atmosphere. The system was cooled to about 0°C and 41.0 mls of 1.6 M butyl lithium solution (in hexane) were then added via a syringe. The system was stirred at 0°C for 5 minutes and then further cooled to −68°C. After cooling, 10.99 gms of t-butyl α-(1,2,4-triazol-1-yl)acetate in tetrahydrofuran were added to the system. The system was stirred at 68°C for 1 hour and then 10.50 gms of 2,4-dichlorobenzaldehyde were slowly added to the system. The system was stirred at −68°C for an additional 10 minutes and then was allowed to warm to room temperature over 45 minutes. Saturated aqueous ammonium chloride was then added to the system and the product was extracted with methylene chloride. The organic solution was washed with water, dried over sodium sulfate and filtered. The solvent was removed by stripping to yield a yellow oil. The oil was taken up in diethyl ether and the product precipitated as a white solid to yield 10.90 gms of the title compound, m.p. 177—182°C.

### Example 3

Bean Powdery Mildew

The compounds of the invention were tested for the control of the Bean Powdery Mildew organism *Erysiphe polygoni*. Seedling bean plants were sprayed with a 250-ppm solution of the test compound in acetone, water and a nonionic emulsifier. The sprayed plants were then inoculated 1 day later with the organism. The plants were maintained for 10 days at temperatures of 68°F (20°C) at night with daytime temperature of 72° to 80°F (22 to 27°C); relative humidity was maintained at 40% to 60%. The percent disease control provided by a given test compound was based on the percent disease reduction relative to the untreated check plants. The results are tabulated in Table II.

### Example 4

Tomato Late Blight

Compounds of the invention were tested for the preventative control of the Tomato Late Blight organism *Phytophthora infestans.* Five- to six-week-old tomato (cultivar Bonny Best) seedlings were used. The tomato plants were sprayed with a 250-ppm suspension of the test compound in acetone, water and a nonionic emulsifier. The sprayed plants were then inoculated 1 day later with the organism, placed in an environmental chamber and incubated at 66° to 68°F (19 to 20°C) and 100% relative humidity for at least 16 hours. Following the incubation, the plants were maintained in a greenhouse for approximately 7 days. The percent disease control provided by a given test compound was based on the percent disease reduction relative to untreated check plants. The results are tabulated in Table II.

Example 5

Celery Late Blight

The Celery Late Blight tests were conducted using celery (Utay) plants 11 weeks old. The Celery Late Blight organism was *Septoria apii.* The celery plants were sprayed with 250-ppm solutions of the candidate toxicant mixed with acetone, water and a nonionic emulsifier. The plants were then inoculated with the organism and placed in an environmental chamber and incubated at 66° to 68°F (19 to 20°C) in 100% relative humidity for an extended period of time (approximatley 48 hours). Following the incubation, the plants were allowed to dry and then were maintained in a greenhouse for approximately 14 days. The percent disease control provided by a given candidate toxicant is based on the percent disease reduction relative to untreated check plants. The results are reported in Table II.

Example 6

Tomato Early Blight

Compounds of the invention were tested for the control of the Tomato Early Blight organism *Alternaria solani conidia.* Tomato (variety Bonny Best) seedlings of 6- to 7-weeks old were used. The tomato plants were sprayed with a 250-ppm solution of the test compound in an acetone-and-water solution containing a small amount of a nonionic emulsifier. The sprayed plants were inoculated 1 day later with the organism, placed in an environmental chamber and incubated at 66° to 68°F (19 to 20°C) and 100% relative humidity for 24 hours. Following the incubation, the plants were maintained in a greenhouse for about 12 days. Percent disease control was based on the percent disease development on untreated check plants. The compounds tested and the results are tabulated in Table II.

Example 7

Grape Downy Mildew

The compounds of the invention were tested for the control of the Grape Downy Mildew organism *Plasmopara viticola.* Detached leaves, between 70 and 85 mm in diameter, from 7-week-old *Vitis vinifera* cultivar Emperor grape seedlings were used as hosts. The leaves were sprayed with a 250-ppm solution of the test compound in acetone. The sprayed leaves were dried, inoculated with a score suspension of the organism, placed in a humid environmental chamber and incubated at 66° to 68°F (19 to 20°C) and about 100% relative humidity. After incubation for 2 days, the plants were then held in a greenhouse 7 to 9 days; then the amount of disease control was determined. The percent disease control provided by a given test compound was based on the percent disease reduction relative to untreated check plants. The results are tabulated in Table II.

Example 8

Leaf Rust

The Leaf Rust test was made using pinto beans. The pathogen was *Uromyces phaseoli tipica.* The pinto bean plants were sprayed with a 250-ppm solution of the test compound in an acetone-water mixture containing a nonionic emulsifier. The treated plants were inoculated thereafter with the pathogen and then incubated in an environmental chamber for approximately 20 hours at 1005 relative humidity and a temperature of 68° to 70°F (20 to 21°C). The plants were then removed from the chamber, allowed to dry, and then maintained in a greenhouse at a 60% to 80% relative humidity. The rate of infection on the leaves was made after about 14 days. The percent disease control provided by a given test compound was based on the percent disease reduction relative to untreated check plants. The results are reported in Table II.

Example 9

Rice Blast

Compounds of this invention were tested for control of the Rice Blast organism *Piricularia oryzae,* using 10- to 14-day-old rice plant seedlings (Calrose M—9 variety). Seedling plants were sprayed with a 625-ppm solution of the test compound in acetone, water and a nonionic emulsifier (ORTHO X—77 spreader). The sprayed plants were inoculated 1 day later with the organism in an environmental chamber. After inoculation, the plants were kept in an environmental chamber for about 48 hours under conditions of about 72° to 75°F (22 to 24°C) and about 100% relative humidity. Following the incubation period, the plants were placed in a greenhouse with a temperature of about 72°F (22°C) and maintained with bottom watering for about 12 to 16 days. The percent disease control provided by a given test compound is based on a comparison of the percentage diseases relative to the percent disease development on the untreated check plants.

$$\% \text{ Control} = 100 \times \frac{(\% \text{ disease in treated plants})}{(\% \text{ disease in check})}$$

The results are tabulated in Table II.

TABLE I

Compounds of the Formula:

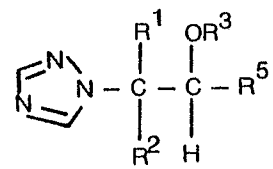

| Compound No. | $R^1$ | $R^2$ | $R^3$ | $R^5$ | ANALYSIS Carbon Calc. | Carbon Found | Hydrogen Calc. | Hydrogen Found | Nitrogen Calc. | Nitrogen Found | Form | m.p. |
|---|---|---|---|---|---|---|---|---|---|---|---|---|
| 1 | 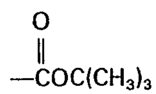 | H | H | 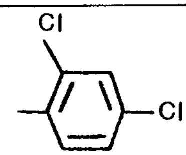 | 50.29 | 50.70 | 4.78 | 5.10 | 11.73 | 12.12 | white solid | 177°—185°C |
| 2 | 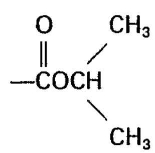 | $—CH_3$ | H | 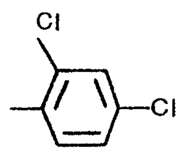 | 48.57 | 50.11 | 4.95 | 4.89 | 12.14 | 12.28 | white solid | 138°—142°C |
| 3 | 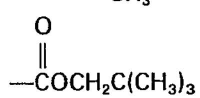 | H | H | 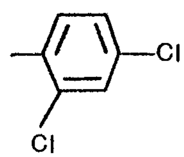 | 51.62 | 52.19 | 5.14 | 5.65 | 11.29 | 10.82 | white solid | 179°—181°C |

TABLE II

Fungicidal Activity
% Control

| Compound No. | GDM | TLB | CLB | TEB | BR | BPM | RB |
|---|---|---|---|---|---|---|---|
| 1 | 0 | 8 | 100 | 98 | 46 | 100 | 0 |
| 2 | 15 | 4 | 92 | 25 | 43 | 100 | 0 |
| 3 | 0 | 0 | 94 | 60 | 13 | 100 | 25 |

GDM — Grape Downy Mildew (*Plasmopara viticola*)
TLB — Tomato Late Blight (*Phytophthora infestans*)
CLB — Celery Late Blight (*Septoria apii*)
TEB — Tomato Early Blight (*Alternaria solani conidia*)
BR — Bean Rust (*Uromyces phaseoli tipica*)
BPM — Bean Powdery Mildew (*Erysiphe polygoni*)
RB — Rice Blast (*Piricularia oryzae*).

**Claims**

1. Compounds represented by the general formula:

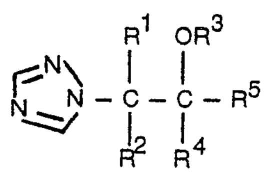

wherein
$R^1$ is isopropoxycarbonyl, t-butoxycarbonyl, or neopentoxycarbonyl;
$R^2$ is hydrogen, methyl or ethyl;
$R^3$ and $R^4$ are hydrogen; and
$R^5$ is 2,4-dihalophenyl,
with the proviso that when $R^1$ is neopentoxycarbonyl, $R^2$ is hydrogen.

2. Compounds as claimed in Claim 1, wherein $R^5$ is 2,4-dichlorophenyl.

3. t-Butyl α-(1,2,4-triazol-1-yl)-β-hydroxy-β-(2,4-dichlorphenyl) propionate.

4. The compound of Claim 1 wherein $R^1$ is isopropoxycarbonyl, $R^2$ is methyl, and $R^5$ is 2,4-dichlorophenyl.

5. The compound of Claim 1 wherein $R^1$ is neopentoxycarbonyl, $R^2$ is hydrogen, and $R^5$ is 2,4-dichlorophenyl.

6. A method of controlling fungi causing fungal plant diseases which comprises contacting said fungi or their plant growth environment with an amount of a compound as claimed in any preceding claim effective to prevent or arrest the growth of said fungi.

7. A fungicidal composition comprising an inert compatible carrier and a fungicidally effective amount of a compound as claimed in any one of Claims 1 to 5.

8. A method of regulating plant growth which comprises treating the growth medium and/or the foliage of plants with a plant growth regulating effective amount of a compound as claimed in any one of Claims 1 to 5.

9. A plant growth regulating composition comprising a plant growth regulating effective amount of a compound as claimed in any one of Claims 1 to 5 and an inert compatible carrier.

**Patentansprüche**

1. Verbindung der allgemeinen Formel

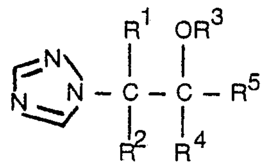

worin $R^1$ Isopropoxycarbonyl, tert.-Butoxycarbonyl oder Neopentoxycarbonyl ist, $R^2$ Wasserstoff, Methyl oder Ethyl bedeutet, $R^3$ und $R^4$ Wasserstoff sind und $R^5$ 2,4-Dihalogenphenyl ist, mit der Ausnahme, daß dann, wenn $R^1$ Neopentoxycarbonyl ist, $R^2$ Wasserstoff ist.

2. Verbindung nach Anspruch 1, worin $R^5$ 2,4-Dichlorphenyl ist.

3. Tert.-Butyl-α-(1,2,4-triazol-1-yl)-β-hydroxy-β-(2,4-dichlorphenyl)-propionat.

4. Verbindung nach Anspruch 1, worin $R^1$ Isopropoxycarbonyl ist, $R^2$ Methyl ist und $R^5$, 2,4-Dichlorphenyl ist.

5. Verbindung nach Anspruch 1, worin $R^1$ Neopentoxycarbonyl ist, $R^2$ Wasserstoff ist und $R^5$ 2,4-Dichlorophenyl ist.

6. Verfahren zur Bekämpfung von Pilzen, welche Pflanzenpilzkrankheiten verursachen, dadurch gekennzeichnet, daß die Pilze oder ihre Pflanzenwachstumsumgebung mit einer Menge einer Verbindung gemäß einem der vorhergehenden Ansprüche kontaktiert wird, die dazu ausreicht, das Wachsen der Pilze zu verhindern oder anzuhalten.

7. Fungizides Mittel aus einem inerten verträglichen Träger und einer fungizid wirksamen Menge einer Verbindung gemäß einem der Ansprüche 1 bis 5.

8. Verfahren zur Steuerung des Pflanzenwachstums, dadurch gekennzeichnet, daß das Wachstumsmedium und/oder die Blätter von Pflanzen mit einer bezüglich der Pflanzenwachstumsregulierung wirksamen Menge einer Verbindung gemäß einem der Ansprüche 1 bis 5 behandelt werden.

9. Pflanzenwachstumsregulierendes Mittel aus einer bezüglich der Pflanzenwachstumsregulierung wirksamen Menge einer Verbindung nach einem der Ansprüche 1 bis 5 und einem inerten verträglichen Träger.

**Revendications**

1. Composés représentés par la formule générale:

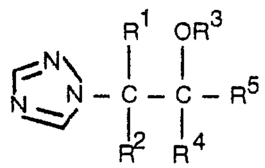

dans laquelle

$R^1$ est un groupe isopropoxycarbonyle, t-butoxycarbonyle ou néopentoxycarbonyle;

$R^2$ est de l'hydrogène, un groupe méthyle ou éthyle;

$R^3$ et $R^4$ sont de l'hydrogène; et

$R^5$ est un groupe 2,4-dihalophényle,

sous réserve que lorsque $R^1$ est un groupe néopentoxycarbonyle, $R^2$ soit un atome d'hydrogène.

2. Composés suivant la revendication 1, dans lesquels $R^5$ est un groupe 2,4-dichlorophényle.

3. α-(1,2,4-triazol-1-yl)-β-hydroxy-β-(2,4-dichlorophényle) propionate de tertio-butyle.

4. Composé suivant la revendication 1, dans lequel $R^1$ est un groupe isopropoxycarbonyle, $R^2$ est un groupe méthyle, et $R^5$ est un groupe 2,4-dichlorophényle.

5. Composé suivant la revendication 1, dans lequel $R^1$ est un groupe néopentoxycarbonyle, $R^2$ est l'hydrogène, et $R^5$ est un groupe 2,4-dichlorophényle.

6. Procédé pour combattre des champignons responsables de maladies fongiques des plantes, qui consiste à faire entrer lesdits champignons ou leur environnement de développement sur les plantes avec une quantité d'un composé suivant l'une quelconque des revendications précédentes, efficace pour prévenir ou arrêter la croissance desdits champignons.

7. Composition fongicide comprenant un support compatible inerte et une quantité efficace du point de vue fongicide d'un composé suivant l'une quelconque des revendications 1 à 5.

8. Procédé pour influencer la croissance de plantes, qui consiste à traiter le milieu de croissance et/ou le feuillage de plantes avec une quantité efficace pour influencer la croissance des plantes d'un composé suivant l'une quelconque des revendications 1 à 5.

9. Composition pour influencer la croissance des plantes, comprenant une quantité efficace pour influencer la croissance des plantes d'une composé suivant l'une quelconque des revendications 1 à 5, et un support compatible inerte.